(19) Europäisches Patentamt
European Patent Office
Office européen des brevets

(11) Veröffentlichungsnummer: **0 043 462**
**B1**

(12) # EUROPÄISCHE PATENTSCHRIFT

(45) Veröffentlichungstag der Patentschrift: **08.05.85**

(21) Anmeldenummer: **81104523.6**

(22) Anmeldetag: **12.06.81**

(51) Int. Cl.[4]: **C 08 G 12/42,** C 07 D 251/64, C 07 D 251/18, C 09 D 3/00, C 09 D 3/50

(54) **Alkoxialkylaminotriazin-Umetherungsprodukte und ihre Verwendung.**

(30) Priorität: **04.07.80 DE 3025352**

(43) Veröffentlichungstag der Anmeldung: **13.01.82 Patentblatt 82/2**

(45) Bekanntmachung des Hinweises auf die Patenterteilung: **08.05.85 Patentblatt 85/19**

(84) Benannte Vertragsstaaten: **AT BE DE FR NL SE**

(56) Entgegenhaltungen:
**DE-A-2 526 874**
**US-A-2 197 357**
**US-A-3 519 627**

(73) Patentinhaber: **BASF Aktiengesellschaft, Carl-Bosch-Strasse 38, D-6700 Ludwigshafen (DE)**

(72) Erfinder: **Hummerich, Rainer, Dr., Budapester Strasse 47, D-6700 Ludwigshafen (DE)**
Erfinder: **Weiss, Wolfram, Dr., Am Speyerweg 40, D-6704 Mutterstadt (DE)**
Erfinder: **Merger, Franz, Dr., Max-Slevogt-Strasse 25, D-6710 Frankenthal (DE)**
Erfinder: **Immel, Guenther, Dr., Mozartstrasse 28, D-6940 Weinheim (DE)**
Erfinder: **Krause, Hans-Joachim, Dr., Pariser Strasse 31, D-6700 Ludwigshafen (DE)**
Erfinder: **Peters, Karl-CLemens, Dr., Otto-Schmidt-Gross-Strasse 4, D-6702 Bad Duerkheim (DE)**

**EP 0 043 462 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft Alkoxialkylaminotriazin-Umetherungsprodukte, die aus Alkoxialkylaminotriazinen und $\beta$-Hydroxialdehyden erhalten werden sowie deren Verwendung als Lackkomponenten.

Alkoxialkylaminotriazine sind seit langem bekannt, beispielsweise Tetramethoxymethylguanamine und Hexamethoxymethylmelamin. Letzteres erhält man z. B. durch Kondensation von 1 Mol Melamin mit 6 Mol Formaldehyd zur Hexamethylolmelamin und anschließende, vollständige Veretherung mit 6 Mol Methanol zum Hexamethoxymethylderivat. Verbindungen dieses Typs sind als hochreaktive Lackharze bekannt und können mit einer Vielzahl Hydroxyl-, Carboxyl- und Amidgruppen-haltiger Polymer vernetzt werden.

Weiter ist bekannt, daß sich solche Verbindungen in Gegenwart saurer Katalysatoren mit anderen Alkoholen umethern lassen und dabei Verbindungen mit zusätzlichen, funktionellen Gruppen entstehen. Derartige Umetherungsreaktionen waren beispielsweise aus folgenden Druckschriften (Alkoholkomponente) bekannt: DE-AS 2 414 426 (Methylglykol), NL-PS 6 614 313 (Hydroxystearinsäure), US-PS 3 519 627 (Dimethylolpropionsäure oder Glyoxylsäure), GB-PS 1 268 481 (Allylalkohol), CH-PS 362 527 (2-Buten-1-ol), US-PS 3 145 207 (Epoxyalkohole) und DE-AS 2 327 147 (Cyclohexanol).

In allen Fällen entstehen Produkte mit neuen, spezifischen Eigenschaften.

Aufgabe der vorliegenden Erfindung ist es, neue Alkoxialkylaminotriazin-Umetherungsprodukte aufzuzeigen, die sich nicht nur sehr vorteilhaft herstellen lassen, sondern auch sehr interessante anwendungstechnische Eigenschaften zeigen.

Gegenstand der vorliegenden Erfindung sind Alkoxialkylaminotriazin-Umetherungsprodukte, die dadurch gekennzeichnet sind, daß sie durch Umsetzung von Alkoxialkylaminotriazinen der allgemeinen Formel (I)

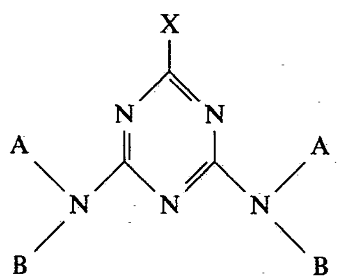

(I)

worin X für Wasserstoff, einen Alkylrest mit 1 bis 15 Kohlenstoffatomen, einen Phenylrest, eine Alkylphenylrest mit 1 bis 4 Kohlenstoffatomen im Alkylrest oder für den Rest

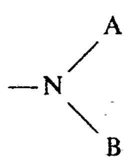

steht; A für Wasserstoff, einen Alkylrest mit 1 bis 4 Kohlenstoffatomen, einen Alkenylrest mit 3 bis 15 Kohlenstoffatomen oder den Rest

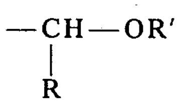

steht, wobei

R und R′ untereinander gleich oder verschieden sind und für Wasserstoff oder eine Alkylgruppe mit 1 bis 9 Kohlenstoffatomen stehen und

B für den Rest

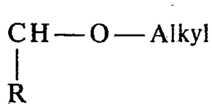

mit 1 bis 9 Kohlenstoffatomen im Alkylrest steht,

mit 0,1 bis 6 Mol, pro Mol Alkoxialkylaminotriazin, eines $\beta$-Hydroxialdehyds der allgemeinen Formel (II)

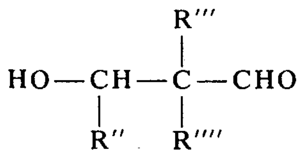

(II)

worin R'', R''' und R'''' untereinander gleich oder verschieden sind und für Wasserstoff, eine Alkylgruppe mit 1 bis 5 Kohlenstoffatomen stehen oder R''' bzw. R'''' zusammen mit dem $\alpha$-Kohlenstoffatom einen Cyclopentan- oder Cyclohexanring bilden sowie R''' und/oder R'''' außerdem für einen Hydroxialkylrest mit 1 bis 4 Kohlenstoffatomen und R'''' für einen Phenylrest stehen können,
in Gegenwart saurer Katalysatoren und Entfernung des bei der Umetherung entstehenden Alkohols erhalten worden sind.

Gegenstand der vorliegenden Erfindung ist außerdem die Verwendung dieser Umetherungsprodukte in Lacken.

Zu den Aufbaukomponenten und zur Herstellung der erfindungsgemäßen Umetherungsprodukte ist im einzelnen folgendes auszuführen.

Als Alkoxialkylaminotriazine der oben genannten allgemeinen Formel (I) kommen beispielsweise entsprechende Derivate des Melamins, Guanamins, Benzoguanoamins und Acetoguanamins in Frage, wie z. B. Hexamethoximethylmelamin, Hexabutoximethylmelamin, Tetramethoxibenzoguanamin, Tetramethoxiacetoguanamin, Tetramethoxi-N,N-dialkylmelamin, N,N',N''-Trimethoxiisobutylmelamin. Geeignet sind vorteilhafterweise auch derartige handelsübliche Produkte, wie z. B. das im technischen Maßstab gewonnene Hexamethoximethylaminotriazin, dessen Methoxigruppen teilweise durch Wasserstoff ersetzt sein können, oder durch weitere niedermolekulare Alkoxigruppen wie z. B. Ethoxi-, Propoxi-, Butoxigruppe ersetzt sein können.

Geeignete $\beta$-Hydroxialdehyde der oben genannten Formel (II) sind im allgemeinen Aldoladditionsprodukte, die gemäß folgendem Reaktionsschema erhältlich sind:

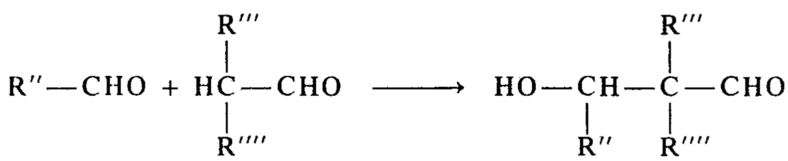

Folgende Aldehyde sind hierfür beispielsweise verwendbar: Formaldehyd, Acetaldehyd, Propanal, n-Butanal, n-Pentanal, n-Hexanal, n-Heptanal, 2-Methylpropanal, 2-Methylbutanal, 2-Methylpentanal, 2-Ethylbutanal, 2-Ethylhexanal, 3-Methylbutanal, Cyclopentyl-carboxaldehyd, Cyclohexyl-carbaldehyd.

Derart erhältliche $\beta$-Hydroxyaldehyde sind z. B. 3-Hydroxibutanal, 3-Hydroxi-2-methyl-propanal, 3-Hydroxi-2-methyl-butanal, 3-Hydroxi-2-methyl-pentanal, 3-Hydroxi-2-methyl-hexanal, 3-Hydroxi-2-methyl-heptanal, 3-Hydroxi-2-ethyl-pentanal, 3-Hydroxi-2-ethyl-hexanal; insbesondere die Umsetzungsprodukte von iso- und n-Alkanalen bzw. Cycloalkyl-carboxaldehyden mit Formaldehyd, z. B. 2,2-Dimethyl-3-hydroxi-propanal, 2-Methyl-2-ethyl-3-hydroxi-propanal, 2-Methyl-2-propyl-3-hydroxi-propanal, 2-Ethyl-2-butyl-3-hydroxipropanal, 2,2-Dimethylol-propanal, 2,2-Dimethylol-butanal, 2,2-Dimethylol-pentanal, 2,2-Dimethylol-3-hydroxi-propanal, 1-Methylol-cyclopentyl-carboxaldehyd, 1-Methylol-cyclohexyl-carboxaldehyd. Verfahren zur Herstellung dieser Produkte sind u. a. beschrieben in DE-AS 1 793 512, DE-OS 1 957 301 und DE-OS 2 507 461. Bevorzugt wird z. B. 2,2-Dimethyl-3-hydroxipropanal (»Hydroxipivalinaldehyd«, R''=H, R'''=R''''=$CH_3$).

Erfindungsgemäß werden pro Mol Alkoxialkylaminotriazin 0,1 bis 6, vorzugsweise 0,5 bis 4,5 Mol des $\beta$-Hydroxialdehyds eingesetzt.

Diese Umsetzung wird in Gegenwart saurer Katalysatoren, wie anorganische, organische Säuren, wie z. B. HCl, $H_2SO_4$, $H_3PO_4$, p-Toluolsulfonsäure, Oxalsäure, Maleinsäure, Maleinsäureanhydrid oder sauren Ionenaustauscher durchgeführt und kann ohne Zusatz eines Lösungsmittels oder in Gegenwart von Lösungsmitteln, wie aliphatischen oder cycloaliphatischen Kohlenwasserstoffen, wie n-Alkane, Cyclohexan, aromatischen Kohlenwasserstoffen, wie Toluol, Xylol, im allgemeinen in einem Temperaturbereich von 50 bis 150°C, vorzugsweise 60 bis 120°C erfolgen.

Zur Herstellung der erfindungsgemäßen Umetherungsprodukte kommen beispielsweise folgende Verfahrensvarianten in Frage:

## Verfahren 1

Ein $\beta$-Hydroxyaldehyd wird in Cyclohexan gelöst. Dann wird durch azeotrope Destillation evetuell vorhandenes Wasser abgetrennt. Zu der Lösung gibt man zum einen das Alkoxialkylaminotriazin und zum anderen die Säure (z. B. p-Toluolsulfonsäure). Anschließend kreist man durch azeotrope Destillation den bei der Umsetzung entstehenden Alkohol aus. Man kühlt auf Raumtemperatur ab und neutralisiert mit der berechneten Menge einer Base (z. B. NaOH). Nun verdünnt man die Reaktionslösung mit z. B. Toluol und filtriert zweckmäßigerweise nach Zugabe einer Filterhilfe mit Hilfe einer Drucknutsche. Das klare Filtrat kann unter Vakuum auf den jeweiligen Feststoffgehalt eingeengt werden.

Verfahren 2

Wie Verfahren 1, jedoch wird als Lösungsmittel direkt Toluol verwendet. Daher wird zum Abtrennen des entstehenden Alkohols Wasser im Abscheidegefäß vorgelegt.

Verfahren 3

(ohne Lösungsmittel)

Ein β-Hydroxyaldehyd wird bei 65°C mit einem Alkoxialkylaminotriazin gemischt. Nach Zugabe einer Säure wird bei einer Temperatur von 80 bis 100°C unter Vakuum der entstehende Alkohol innerhalb von 30 bis 180 Minuten abdestilliert. Nun wird das Reaktionsgemisch mit einem Lösungsmittel (z. B. Toluol, Xylol) verdünnt. Nach Neutralisation mit der berechneten Menge einer Base (wie z. B. NaOH) wird vom Salz abfiltriert. Das klare Filtrat kann auf jeden gewünschten Feststoffgehalt eingestellt werden.

Mit den erfindungsgemäßen Alkoxialkylaminotriazin-Umetherungsprodukten lassen sich auch sehr vorteilhafte Folgeprodukte herstellen, die z. B. durch chemische Modifizierung der Aldehydgruppe in (I) wie Oxidation, Reduktion, Disproportionierung und Kondensation mit Nucleophilen entstehen. Die Folgereaktion kann dabei während der Umetherungsreaktion ablaufen oder in einem getrennten, der Umetherung nachgeschalteten Verfahrensschritt erfolgen.

Die erfindungsgemäßen Produkte erwiesen sich sowohl in säurehärtenden Lacken als auch in Einbrennlacken als Lackkomponenten, z. B. als Bindemittel mit überraschend guten Eigenschaften.

Die Herstellung der erfindungsgemäßen Produkte und deren Verwendung soll im folgenden an Beispielen näher erläutert werden.

Die in den Beispielen genannten Teile und Prozente sind — soweit nicht anders angegeben — Gewichtsteile bzw. Gewichtsprozente.

Herstellung der β-Hydroxialdehyde

Die Herstellung der zu verwendenden β-Hydroxialdehyde kann u. a. nach DE-OS 1 957 301, DE-OS 2 507 461 und DE-AS 1 793 512 durchgeführt werden. Besonders vorteilhaft für die Umsetzungen mit Alkoxialkylaminotriazinen ist die in situ Herstellung der β-Hydroxialdehyde wie folgt:

734,7 Teile Isobutyraldehyd werden unter Rühren mit 750 Teilen 40%igem Formaldehyd gemischt. Nachdem die Mischung auf ca. 40°C erwärmt wurde, läßt man 25,7 Teile 40%iges Trimethylamin zulaufen. Man läßt die Temperatur bis auf 45°C ansteigen und kühlt dann so, daß die Temperatur nur noch langsam auf 60°C ansteigt. Nun gibt man weitere 8,6 Teile 40%iges Trimethylamin dazu. Die Reaktion wird nun so gesteuert, daß die Temperatur nur langsam auf 75 bis 80°C ansteigt, die Lösung dabei aber nicht zum Sieden kommt. Anschließend wird kurz auf 90 bis 92°C erwärmt und sofort wieder auf 65 bis 70°C abgekühlt. Dann engt man die Reaktionslösung bei einer Innentemperatur 65°C unter Wasserstrahlvakuum solange ein, bis die ersten Tropfen Hydroxypivalinalaldehyd übergehen. Dies ist bei einer Sumpftemperatur von 90°C, einer Siedetemperatur von 75°C bei vollem Wasserstrahlvakuum der Fall.

Der so erhaltene β-Hydroxialdehyd wird dann nach einem der oben genannten drei Verfahren mit einem Alkoxialkylaminotriazin umgesetzt, wobei die Umsetzung sowohl im gleichen Reaktionsgefäß als auch in einem davon getrennten durchgeführt werden kann.

Beispiele 1 bis 41

Die in den Tabellen angegebenen Feststoffgehalte und Viskositäten wurden folgendermaßen bestimmt:

Feststoffgehalt
Es werden 2 g Harzlösung 2 Stunden bei 120°C im Umlufttrockenschrank getrocknet und dann erneut gewogen.

Viskosität
Es wurde die dynamische Viskosität bei 20°C mit einem Rotationsviskosimeter (z. B. Rotavisko der Firma Haake) bestimmt.

HMMM = Hexamethoximethylmelamin
HPA = Hydroxypivalinaldehyd

Tabelle 1

Umsetzungen von HMMM mit 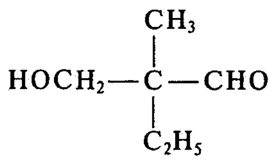 (I) bzw. 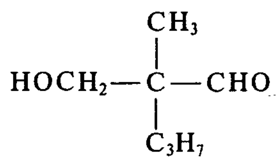 (II)

| Beispiel Nr. | Verfahren | Alkoxyalkylaminotriazin | Teile | β-Hydroxy-aldehyd | Teile |
|---|---|---|---|---|---|
| 1 | 1 | 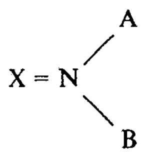 A = B = $CH_2-OCH_3$ = MMM | 195 | I | 232 |
| 2 | 1 | HMMM | 260 | I | 232 |
| 3 | 1 | HMMM | 312 | I | 232 |
| 4 | 1 | HMMM | 390 | I | 232 |
| 5 | 1 | HMMM | 180 | II | 240 |
| 6 | 1 | HMMM | 240 | II | 240 |
| 7 | 1 | HMMM | 288 | II | 240 |
| 8 | 1 | HMMM | 360 | II | 240 |
| 9 | 1 | HMMM | 195 | II | 260 |

Tabelle 1 (Fortsetzung)

| Beispiel Nr | Säure | Teile | Beschreibung des Produktes Aussehen, Feststoffgehalt; | Viskosität bei 20°C |
|---|---|---|---|---|
| 1 | p-Toluolsulfonäure | 5 | klare Lösung, 59,6%, | 18 mPas |
| 2 | p-Toluolsulfonäure | 5 | klare Lösung, 59,4%, | 28 mPas |
| 3 | p-Toluolsulfonäure | 5 | klare Lösung, 68,3%, | 26 mPas |
| 4 | p-Toluolsulfonäure | 5 | klare Lösung, 64,3%, | 32 mPas |
| 5 | p-Toluolsulfonäure | 4,6 | klare Lösung, 67,9%, | 12 mPas |
| 6 | p-Toluolsulfonäure | 4,6 | klare Lösung, 71%, | 17 mPas |
| 7 | p-Toluolsulfonäure | 4,6 | klare Lösung, 71%, | 19 mPas |
| 8 | p-Toluolsulfonäure | 4,6 | klare Lösung, 73,6%, | 24 mPas |
| 9 | p-Toluolsulfonäure | 5 | klare Lösung, 60%, | 1700 mPas |

Tabelle 2

Umsetzungen von HMMM mit wäßriger Lösung von HPA

| Bsp. Nr. | Verfahren | Alkoxyalkylaminotriazin | Teile | β-Hydroxyaldehyd | Teile |
|---|---|---|---|---|---|
| 10 | 1 | 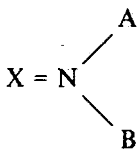 A = B = $CH_2OCH_3$ = HMMM | 772 | 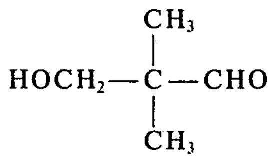 = HPA (72,1%ig) | 140 |
| 11 | 1 | HMMM | 772 | HPA (72,1%ig) | 280 |
| 12 | 1 | HMMM | 397 | HPA (83,2%ig) | 250 |
| 13 | 1 | HMMM | 318 | HPA (83,2%ig) | 250 |
| 14 | 1 | HMMM | 265 | HPA (83,2%ig) | 250 |
| 15 | 2 | HMMM | 841 | HPA (78,6%ig) | 140 |
| 16 | 2 | HMMM | 841 | HPA (78,6%ig) | 280 |
| 17 | 2 | HMMM | 420 | HPA (78,6%ig) | 280 |

Tabelle 2 (Fortsetzung)

| Beispiel Nr | Säure | Teile | Beschreibung des Produktes Aussehen, Feststoffgehalt |
|---|---|---|---|
| 10 | p-Toluolsulfonsäure | 5 | klare Lösung, 47,7% in 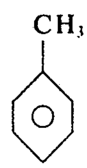 |
| 11 | p-Toluolsulfonsäure | 10 | klare Lösung, 51,74% in 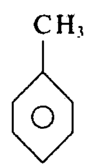 |
| 12 | p-Toluolsulfonsäure | 10 | klare Lösung, 50,2% in 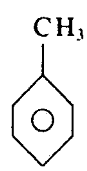 |
| 13 | p-Toluolsulfonsäure | 10 | klare Lösung, 50,22% in 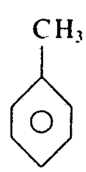 |

Fortsetzung

| Beispiel Nr | Säure | Teile | Beschreibung des Produktes Aussehen, Feststoffgehalt |
|---|---|---|---|
| 14 | p-Toluolsulfonsäure | 10 | klare Lösung, 53,95% in 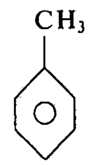 |
| 15 | p-Toluolsulfonsäure | 5 | klare Lösung, 49,3% |
| 16 | p-Toluolsulfonsäure | 10 | klare Lösung, 49,89% |
| 17 | p-Toluolsulfonsäure | 10 | klare Lösung, 50,92% |

Tabelle 3

Umsetzungen von Tetrakismethoxymethylbenzoguanamin (III)

| Bsp. Nr. | Verfahren | Alkoxyalkylaminotriazin | Teile | β-Hydroxyaldehyd | Teile |
|---|---|---|---|---|---|
| 18 | 1 | X = Phenyl; A = B = $CH_2OCH_3$ = III | 92,5 | 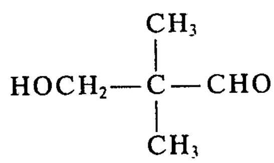 (HPA) (77%ig) | 135 |
| 19 | 1 | III | 185 | HPA (77%ig) | 135 |
| 20 | 1 | III | 123 | HPA (77%ig) | 135 |
| 21 | 2 | III | 181,5 | 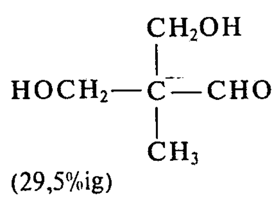 (29,5%ig) | 200 |
| 22 | 3 | III | 302 | 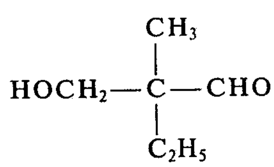 | 290 |
| 23 | 3 | III | 227 | desgl. | 290 |
| 24 | 3 | III | 302 | 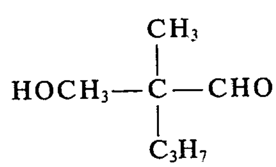 | 325 |
| 25 | 3 | III | 227 | desgl. | 304 |

Tabelle 3 (Fortsetzung)

| Bsp. Nr | Säure | Teile | Beschreibung des Produktes Aussehen, Feststoffgehalt; | | Viskosität bei 30°C |
|---|---|---|---|---|---|
| 18 | p-Toluolsulfonäure | 2,5 | klare Lösung, | 48%ig in Toluol | |
| 19 | p-Toluolsulfonäure | 2,5 | klare Lösung, (toluol. Lösung) | 63,4%ig | 220 mPas |
| 20 | p-Toluolsulfonäure | 2,5 | klare Lösung, | 32,9%ig in Cyclohexan | |
| 21 | p-Toluolsulfonäure | 1,125 | klare Lösung, | 56,1%ig in Toluol | |
| 22 | p-Toluolsulfonäure | 6,25 | | 76,4% | 732 mPas |
| 23 | p-Toluolsulfonäure | 6,25 | | 69,4% | 304 mPas |
| 24 | p-Toluolsulfonäure | 6,25 | | 69,4% | 165 mPas |
| 25 | p-Toluolsulfonäure | 6,25 | | 67,7% | 347 mPas |

Tabelle 4

Variationen des Verhältnisses HMMM : HPA

| Bsp. Nr. | Ver-fahren | Alkoxyalkylaminotriazin | Teile | β-Hydroxyaldehyd | Teile |
|---|---|---|---|---|---|
| 26 | 3 | 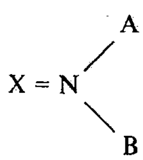 A = B = $CH_2OCH_3$ = HMMM | 1040 | 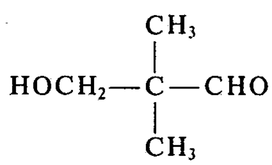 (HPA) | 408 |
| 27 | 3 | HMMM | 891 | HPA | 408 |
| 28 | 3 | HMMM | 693 | HPA | 408 |
| 29 | 3 | HMMM | 624 | HPA | 408 |
| 30 | 3 | HMMM | 780 | HPA | 408 |
| 31 | 3 | HMMM | 1040 | HPA | 408 |

Tabelle 4 (Fortsetzung)

| Beispiel Nr. | Säure | Teile | Beschreibung des Produktes Aussehen, Feststoffgehalt; | Viskosität bei 30°C |
|---|---|---|---|---|
| 26 | p-Toluolsulfonäure | 10 | klare Lösung, 85%, | 22 224 mPas |
| 27 | p-Toluolsulfonäure | 10 | klare Lösung, 81,2%, | 4 145 mPas |

Fortsetzung

| Beispiel Nr. | Säure | Teile | Beschreibung des Produktes Aussehen, Feststoffgehalt; | Viskosität bei 30°C |
|---|---|---|---|---|
| 28 | p-Toluolsulfonäure | 10 | klare Lösung, 80,8%, | 7 086 mPas |
| 29 | p-Toluolsulfonäure | 10 | klare Lösung, 86,4%, | 30 337 mPas |
| 30 | p-Toluolsulfonäure | 10 | klare Lösung, 71,4%, | 2 161 mPas |
| 31 | p-Toluolsulfonäure | 10 | klare Lösung, 72,6% | 420 mPas |

Tabelle 5

Umsetzungen von HMMM mit verschiedenen β-Hydroxyaldehyden

| Bsp. Nr. | Ver-fahren | Alkoxyalkylaminotriazin | Teile | β-Hydroxyaldehyd | Teile |
|---|---|---|---|---|---|
| 32 | 3 | 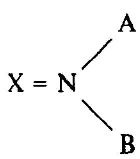 A = B = $CH_2OCH_3$ = HMMM | 1040 | 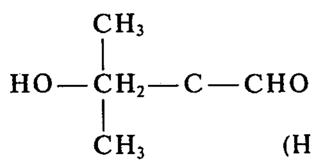 (HPA) | 408 |
| 33 | 3 | HMMM | 780 | HPA | 408 |
| 34 | 3 | HMMM | 195 | 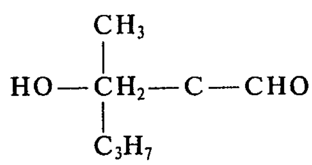 | 260 |
| 35 | 3 | HMMM | 260 | desgl. | 260 |
| 36 | 3 | HMMM | 520 | 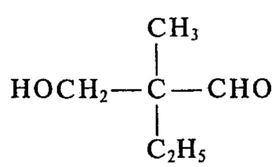 | 464 |
| 37 | 3 | HMMM | 780 | desgl. | 928 |
| 38 | 2 | HMMM | 780 | 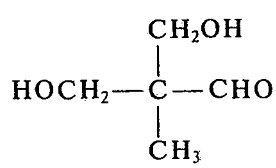 (29,5%ig) | 400 |
| 39 | 2 | HMMM | 520 | desgl. | 800 |
| 40 | 2 | HMMM | 260 | HPA | 204 |
| 41 | 2 | HMMM | 390 | HPA | 204 |

Tabelle 5 (Fortsetzung)

| Beispiel Nr. | Säure | Teile | Beschreibung des Produktes Aussehen, Feststoffgehalt | Viskosität bei 20 bzw. 30°C |
|---|---|---|---|---|
| 32 | p-Toluolsulfonsäure | 10 | klare Lösung, 85%, | 22 224 mPas (bei 30°C) |
| 33 | p-Toluolsulfonsäure | 10 | klare Lösung, 85,1%, | 12 051 mPas (bei 30°C) |
| 34 | p-Toluolsulfonsäure | 5 | klare Lösung, 76,7%, | 6 120 mPas (bei 20°C) |
| 35 | p-Toluolsulfonsäure | 5 | klare Lösung, 73,6%, | 4 800 mPas (bei 20°C) |
| 36 | p-Toluolsulfonsäure | 10 | klare Lösung, 73,9%, | 2 000 mPas (bei 20°C) |
| 37 | p-Toluolsulfonsäure | 20 | klare Lösung, 74,8%, | 7 532 mPas (bei 30°C) |
| 38 | p-Toluolsulfonsäure | 2,25 | klare Lösung, 57,4%, | |
| 39 | p-Toluolsulfonsäure | 5 | klare Lösung, 51,2%, | 400 mPas (bei 20°C) |
| 40 | p-Toluolsulfonsäure | 5 | klare Lösung, 68,4%, | |
| 41 | p-Toluolsulfonsäure | 5 | klare Lösung, 70,9%, | 420 mPas (bei 30°C) |

Im folgenden sind anwendungstechnische Prüfungen mit einigen Harzlösungen der Beispiele aus den Tabellen 1 bis 5 aufgeführt.

## I. Einbrennlacke

Der Vorteil der höheren Elastizität (=Erichsentiefung) gegenüber handelsüblichen MF-Harzen bei gleicher Härte wird an den Beispielen 41 bis 51 gezeigt.
Als handelsübliches Alkoxialkylaminotriazin-Harz wurde ein butanolverethertes hochreaktives Melamin/Formaldehyd-Harz, ca. 60%ig in Butanol/Xylol eingesetzt.

### Beispiel 42

Die nach Beispiel 40 hergestellte Harzlösung wurde als Härtungsmittel für einen Lack eingesetzt, dessen Basisharz ein polymeres Acrylatharz der Zusammensetzung 46 Teile Styrol, 37 Teile Butylacrylat, 15 Teile Hydroxipropylacrylat, 2 Teile Acrylsäure ist (als 60%ige Lösung in Xylol/Butanol = 8 : 2 vorliegend). Das Verhältnis Acrylatharz : Aminoharz ist 7 : 3, fest auf fest gerechnet.

Rezept A

58,3 Teile Acrylatharz [60%ig in Xylol/Butanol (8 : 2)]
21,9 Teile Harzlösung nach Beispiel 40 (68,4%ig in Xylol)
50 Teile $TiO_2$ (®Kronos RN 57 der Firma Titangesellschaft Leverkusen)
20 Teile Xylol/Ethylglykol

werden mit ca. 100 Teilen Glaskugeln ca. 20 Minuten angerieben. Als Mahlaggregat wurde dabei ein Gerät mit intensiver dreidimensionaler Schüttelbewegung verwendet. (»Red Devil«, Hersteller: Red Devil Inc., New Jersey.) Die Lacklösung wird von den Kugeln abgesiebt und mit Xylol/Ethylglykol auf 20 Sekunden Auslaufzeit [DIN-Becher, 4 mm Düse] verdünnt. Dieser Lack wird mit der Spritzpistole auf ein 1 mm starkes Tiefziehblech aufgetragen (Schichtdicke ca. 50 μm trocken). Nach dem Spritzen wird das beschichtete Blech 5 Minuten abgelüftet und dann bei 150°C im Umluftofen 30 Minuten lang eingebrannt.

Prüfergebnisse

| | |
|---|---|
| Glanz (nach Gardner, 60°) | 90% |
| Erichsentiefung | 6,2 mm (DIN 53156) |

| | |
|---|---|
| Pendeldämpfung (n. König) | 182 s (DIN 53157) |
| Gitterschnittprüfung | 2,5 (DIN 53151) |

Der Vergleichsversuch mit dem handelsüblichen Melamin-Formaldehyd-Harz ergab unter gleichen Bedingungen:

| | |
|---|---|
| Glanz | 83% |
| Erichsentiefung | 2,6 mm |
| Pendeldämpfung | 157 s |
| Gitterschnittprüfung | 3 |

Beispiel 43

Das gleiche Ausgangsharz (siehe Beispiel 40) wird in einem modifizierten Rezept angewandt; wobei das Verhältnis Acrylatharz : Aminoharz = 1 : 1 (fest auf fest gerechnet) beträgt:

Rezept B

41,7 Teile Acrylatharz (60%ig in Xylol)
36,5 Teile Harzlösung nach Beispiel 40 (68,4%ig in Xylol)
50 Teile $TiO_2$ (RN 57)
20 Teile Xylol/Ethylglykol

werden mit 100 Teilen Glaskugeln abgerieben (wie dies oben beschrieben wurde) und weiter verarbeitet.

| Prüfergebnisse | mit erfindungsgemäßem Produkt | mit handelsüblichem Harz |
|---|---|---|
| Glanz | 88 | 83 |
| Erichsentiefung | 5,2 | 1,5 |
| Pendeldämpfung | 179 | 162 |
| Gitterschnittprüfung | 3 | 4 |

Beispiel 44

Mit der nach Beispiel 30 hergestellten Harzlösung wurde nach dem Rezept B (also Verhältnis Acrylat : MF-Harz = 1 : 1) ein Lack hergestellt und in der beschriebenen Weise appliziert und eingebrannt.

| Prüfergebnisse | mit erfindungsgemäßem Produkt | mit handelsüblichem Harz |
|---|---|---|
| Glanz | 88 | 90 |
| Erichsentiefung | 5,2 | 1,2 |
| Pendeldämpfung | 188 | 165 |
| Gitterschnittprüfung | 2,5 | 4 |

Beispiel 45

Mit der nach Beispiel 30 hergestellten Harzlösung wurde nach dem Rezept B ein Lack auf der Basis eines synthetischen Fettsäurealkydharzes (z. B. ®Alkydal F 251 der Firma Bayer, Leverkusen) hergestellt. Das Acrylatharz im Rezept B ist also durch ®Alkydal ersetzt worden.

| Prüfergebnisse | mit Harz nach Beispiel 30 | mit handelsüblichem Harz |
|---|---|---|
| Glanz | 94 | 60 |
| Erichsentiefung | 4,0 | 0,6 |
| Pendeldämpfung | 193 | 171 |
| Gitterschnittprüfung | 3 | 4 |

Beispiel 46

Es wird wie in Beispiel 43 beschrieben verfahren, jedoch mit Harz aus Beispiel 31.

| Prüfergebnisse | mit erfindungsgemäßem Produkt | mit handelsüblichem Harz |
|---|---|---|
| Glanz | 86 | 90 |
| Erichsentiefung | 6,5 | 1,2 |
| Pendeldämpfung | 167 | 165 |
| Gitterschnittprüfung | 2 | 4 |

Beispiel 47

Es wird wie in Beispiel 45 verfahren, jedoch mit dem Harz von Beispiel 31.

| Prüfergebnisse | mit erfindungsgemäßem Produkt | mit handelsüblichem Harz |
|---|---|---|
| Glanz | 93 | 60 |
| Erichsentiefung | 7,3 | 0,6 |
| Pendeldämpfung | 170 | 171 |
| Gitterschnittprüfung | 2 | 4 |

Beispiel 48

Es wird wie in Beispiel 43 verfahren, jedoch mit dem Harz von Beispiel 1.

| Prüfergebnisse | mit erfindungsgemäßem Produkt | mit handelsüblichem Harz |
|---|---|---|
| Glanz | 81 | 83 |
| Erichsentiefung | 5,1 | 1,5 |
| Pendeldämpfung | 186 | 162 |
| Gitterschnittprüfung | 3 | 4 |

Beispiel 49

Es wird wie in Beispiel 43 beschrieben gearbeitet, jedoch mit dem Harz von Beispiel 4.

| Prüfergebnisse | mit erfindungsgemäßem Produkt | mit handelsüblichem Harz |
|---|---|---|
| Glanz | 83 | 83 |
| Erichsentiefung | 6,5 | 1,5 |
| Pendeldämpfung | 181 | 162 |
| Gitterschnittprüfung | 3 | 4 |

Beispiel 50

Wie Beispiel 43, jedoch mit dem Harz von Beispiel 6.

Beispiel 51

Wie Beispiel 43, jedoch mit dem Harz von Beispiel 18.

| Prüfergebnisse | Beispiel 51 | Beispiel 50 | handelsübliches Harz |
|---|---|---|---|
| Glanz | 86 | 86 | 83 |
| Erichsentiefung | 4,5 | 3,2 | 1,5 |
| Pendeldämpfung | 178 | 179 | 162 |
| Gitterschnittprüfung | 2 | 3 | 4 |

II. SH-Lacke (säurehärtende Lacke)

Harnstoff-Harze eignen sich im allgemeinen zur Verwendung in SH-Lacken und in Einbrennlacken. Bei Melamin/Formaldehyd-Harzen sind die hochmethylolierten und weitgehend veretherten Typen in Einbrennlacken wenig reaktiv, jedoch für SH-Lacke geeignet.

Umgekehrt sind die mit weniger Formaldehyd hergestellten und nur teilweise veretherten MF-Harze für Einbrennsysteme geeignet, aber in SH-Lacken nicht brauchbar.

Die in den Beispielen beschriebenen Harze eignen sich dagegen sowohl für Einbrenn- als auch für SH-Lacke, wie aus den folgenden Beispielen zu ersehen ist. Die Lacke für diese Beispiele wurden nach folgenden allgemeinen Rezepten hergestellt.

Rezept C

- 50 Teile Acrylatharz (60%ig in Butanol/Xylol)
- 29,2 Teile eines erfindungsgemäßen Alkoxialkylaminotriazin-Harzes oder eines Handelsproduktes (68,4%ig in Xylol)
- 50 Teile $TiO_2$ (RN 57)
- 20 Teile Xylol/Butanol

Acrylatharz und Aminoharz werden also im Verhältnis 6 : 4 (fest/fest) eingesetzt und es wird mit 100% $TiO_2$ (berechnet auf Bindemitteln) pigmentiert. Verarbeitung wie beim Rezept A beschrieben.

Rezept D

- 50 Teile Ricinenalkyd (60%ig in Xylol)
- 29,2 Teile eines erfindungsgemäßen Alkoxialkylaminotriazin-Harzes oder eines Handelsproduktes (68,4%ig in Xylol)
- 50 Teile $TiO_2$ (RN 57)
- 20 Teile Xylol/Butanol

Das Verhältnis Ricinenalkyd : Aminoharz beträgt 6 : 4 (fest auf fest gerechnet). Pigmentiert ist mit 100% $TiO_2$, berechnet auf Bindemittel. Verarbeitung wie beim Rezept A beschrieben.

Rezept E (für Säurehärtung)

35 Teile Ricinenalkyd (60%ig in Xylol)
13,9 Teile eines erfindungsgemäßen Alkoxialkylaminotriazin-Harzes bzw. eines Handelsproduktes (69%ig)
22,1 Teile Ethanol/Ethylglykol 2 : 1
3 Teile p-Toluolsulfonsäure (20%ig in Ethanol), d. h. es werden 6% Säure auf Aminoharz (fest) zugesetzt.

Die Bestandteile des SH-Lackes werden bis zur homogenen Mischung gerührt und zum Schluß die Säurelösung eingearbeitet. Die Lacke werden mit einem Filmziehgerät 150 µm naß auf Glas aufgezogen und 30 Minuten bei 40°C gehalten. Bis zur Prüfung der Pendeldämpfung nach König werden die Prüfplatten 24 Stunden im Normklima bei 23°C gelagert.

Prüfergebnisse: Pendeldämpfung nach König (in Sekunden)

| verwendetes Harz | Rezept C | Rezept D | Rezept E SH-Lacke |
|---|---|---|---|
| Beispiel 52: nach Beispiel 40 | 182 | 198 | 171 |
| Beispiel 53: nach Beispiel 1 | 174 | 179 | 157 |
| Beispiel 54: nach Beispiel 5 | 171 | 182 | 120 |
| Beispiel 55: nach Beispiel 14 | 186 | 197 | 155 |
| Beispiel 56: nach Beispiel 13 | 185 | 197 | 174 |
| handelsübliches SH-Melamin-Harz | 38 | 28 | 150 |
| handelsübliches Einbrenn-Melamin-Harz | 162 | 175 | 40 |

Als Maß für die Härtungsgeschwindigkeit bei säurehärtenden Lacken kann an Stelle der Pendeldämpfung auch der sogenannte Kempf-Test in Anlehnung an DIN 53 159 mit Hilfe des Abkreide-Stempelgerätes nach Kempf (Fa. Erichsen GmbH) genommen werden. Dies führt zu dem gleichen Ergebnis. Für den Kempf-Test wurde mit folgendem Lackrezept gearbeitet:

Rezept F

70 Teile Ricinenalkyd (60%ig)
19 Teile (fest) eines erfindungsgemäßen Alkoxialkylaminotriazin-Harzes oder eines Handelsproduktes
53 Teile Ethanol/Ethylglykol 2 : 1
12 Teile p-Toluolsulfonsäure (10%ig in Ethanol)

Verarbeitung wie oben angegeben.

Prüfergebnisse

| Beispiel | verwendetes Harz | Zyklus-Zeit bei 40°C | | | | | |
|---|---|---|---|---|---|---|---|
| | | 5′ | 10′ | 15′ | 20′ | 25′ | 30′ |
| 57 | nach Beispiel 40 | 3–4 | 2 | 0 | 0 | 0 | 0 |
| 58 | nach Beispiel 1 | 1–2 | 0 | 0 | 0 | 0 | 0 |
| 59 | nach Beispiel 5 | 2–3 | 0–1 | 0 | 0 | 0 | 0 |

Fortsetzung

| Beispiel | verwendetes Harz | Zyklus-Zeit bei 40°C | | | | | |
|---|---|---|---|---|---|---|---|
| | | 5′ | 10′ | 15′ | 20′ | 25′ | 30′ |
| 60 | nach Beispiel 14 | 1–2–A | 0 | 0 | 0 | 0 | 0 |
| 61 | nach Beispiel 13 | 1 | 0 | 0 | 0 | 0 | 0 |
| Vergleiche | | | | | | | |
| handelsübliches SH-MF-Harz | | 4 | 4 | 3–4 | 3 | 2–3 | 2 |
| handelsübliches Einbrenn-MF-Harz | | 5 | 5 | 5 | 5 | 5 | 5 |
| handelsübliches SH-HF-Harz | | 1–2 | A | 0 | 0 | 0 | 0 |

0 = klebfrei
5 = noch stark klebrig
MF-Harz = Melamin-Formaldehyd-Harz
SH-MF-Harz = säurehärtbares Melamin-Formaldehydharz
SH-HF-Harz = säurehärtbares Harnstoff-Formaldehydharz

**Patentansprüche**

1. Alkoxialkylaminotriazin-Umetherungsprodukte, dadurch gekennzeichnet, daß sie durch Umsetzung von Alkoxialkylaminotriazinen der allgemeinen Formel (I)

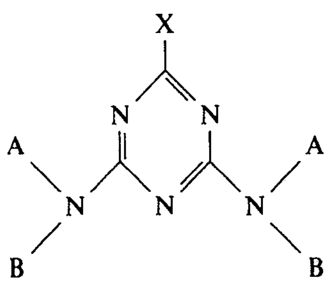

(I)

worin X für Wasserstoff, einen Alkylrest mit 1 bis 15 Kohlenstoffatomen, einen Phenylrest, einen Alkylphenylrest mit 1 bis 4 Kohlenstoffatomen im Alkylrest oder für den Rest

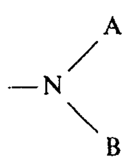

steht; A für Wasserstoff, einen Alkylrest mit 1 bis 4 Kohlenstoffatomen, einen Alkenylrest mit 3 bis 15 Kohlenstoffatomen oder den Rest

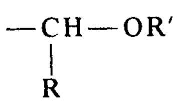

steht, wobei
R und R′ untereinander gleich oder verschieden sind und für Wasserstoff oder eine Alkylgruppe mit 1 bis 9 Kohlenstoffatomen stehen und
B für den Rest

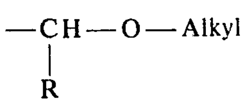

mit 1 bis 9 Kohlenstoffatomen im Alkylrest steht, mit 0,1 bis 6 Mol, pro Mol Alkoxialkylaminotriazin, eines $\beta$-Hydroxialdehyds der allgemeinen Formel (II)

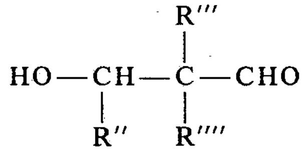

(II)

worin R'', R''' und R'''' untereinander gleich oder verschieden sind und für Wasserstoff, eine Alkylgruppe mit 1 bis 5 Kohlenstoffatomen stehen oder R''' bzw. R'''' zusammen mit dem $\alpha$-Kohlenstoffatom einen Cyclopentan- oder Cyclohexanring bilden sowie R''' und/oder R'''' außerdem für einen Hydroxialkylrest mit 1 bis 4 Kohlenstoffatomen und R'''' für einen Phenylrest stehen können, in Gegenwart saurer Katalysatoren und Entfernung des bei der Umetherung entstehenden Alkohols erhalten worden sind.

2. Alkoxialkylaminotriazin-Umetherungsprodukte nach Anspruch 1, dadurch gekennzeichnet, daß als Alkoxialkylaminotriazin im wesentlichen Hexamethoximethylaminotriazin verwendet wird.

3. Alkoxialkylaminotriazin-Umetherungsprodukte nach Anspruch 1 oder 2, dadurch gekennzeichnet, daß als $\beta$-Hydroxialdehyd Hydroxipivalaldehyd verwendet wird.

4. Alkoxialkylaminotriazin-Umetherungsprodukte nach einem der vorhergehenden Ansprüche, dadurch gekennzeichnet, daß sie durch Umsetzung von Alkoxialkylaminotriazinen der allgemeinen Formel (I) mit 0,5 bis 4,5 Mol, pro Mol Alkoxialkylaminotriazin, eines $\beta$-Hydroxialdehyds der allgemeinen Formel (II) erhalten worden sind.

5. Verwendung der Alkoxialkylaminotriazin-Umetherungsprodukte nach einem der Ansprüche 1 bis 4 in Lacken.

## Claims

1. An alkoxyalkylaminotriazine trans-etherification product which has been obtained by reacting an alkoxyalkylaminotriazine of the general formula (I)

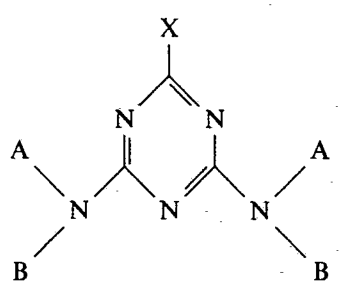

(I)

where X is hydrogen, alkyl of 1 to 15 carbon atoms, phenyl, alkylphenyl, where alkyl is of 1 to 4 carbon atoms, or

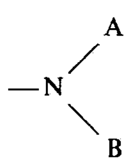

A is hydrogen, alkyl of 1 to 4 carbon atoms, alkenyl of 3 to 15 carbon atoms or

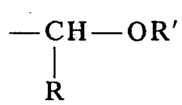

R ans R' being identical or different and each being hydrogen or alkyl of 1 to 9 carbon atoms, and B is

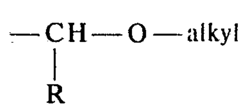

where alkyl is of 1 to 9 carbon atoms, with from 0,1 to 6 moles, per mole of alkoxyalkylaminotriazine, of a $\beta$-hydroxyaldehyde of the general formula (II)

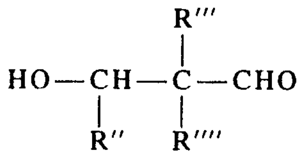

(II)

where R″, R‴ and R⁗ are identical or different and each is hydrogen or alkyl of 1 to 5 carbon atoms, or R‴ or R⁗ together with the $\alpha$-carbon atom form a cyclopentane or cyclohexane ring, and R‴ and/or R⁗ can also be hydroxyalkyl of 1 to 4 carbon atoms and R⁗ can also be phenyl, in the presence of an acidic catalyst, and removing the alcohol resulting from the trans-etherification.

2. An alkoxyalkylaminotriazine trans-etherification product as claimed in claim 1, wherein the alkoxyalkylaminotriazine used is essentially hexamethoxymethylaminotriazine.

3. An alkoxyalkylaminotriazine trans-etherification product as claimed in claim 1 or 2, wherein the $\beta$-hydroxyaldehyde used is hydroxypivalaldehyde.

4. An alkoxyalkylaminotriazine trans-etherification product as claimed in any of the preceding claims, which has been obtained by reacting an alkoxyalkylaminotriazine of the general formula (I) with from 0.5 to 4.5 moles, per mole of alkoxyalkylaminotriazine, of a $\beta$-hydroxyaldehyde of the general formula (II).

5. The use of an alkoxyalkylaminotriazine trans-etherification product as claimed in any of claims 1 to 4 in paints.

**Revendications**

1. Produits de transéthérification d'alcoxyalkylamino-triazines, caractérisés en ce qu'ils ont été préparés par la réaction d'alcoxyalkyl-amino-triazines de la formule générale (I)

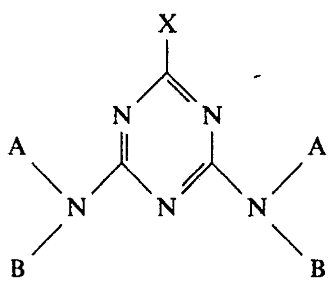

(I)

dans laquelle

X désigne un atome d'hydrogène, un radical alkyle en $C_1$ à $C_{15}$, un groupe phényle, un groupe alkyl(en $C_1$ à $C_4$)-phényle ou un groupe

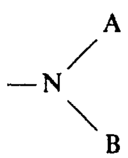

A représente un atome d'hydrogene, un radical alkyle en $C_1$ à $C_4$, un radical alcényle en $C_3$ à $C_{15}$ ou un groupe

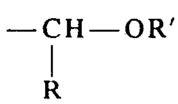

dans lequel R et R', qui peuvent être identiques ou différents, désignent chacun un atome d'hydrogène ou un radical alkyle en $C_1$ à $C_9$, et

B représente un groupe

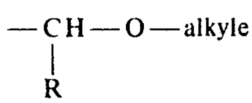

alkyle avec un radical alkyle en $C_1$ à $C_9$,

en présence de catalyseurs acides avec 0,1 à 6 moles par mole d'alcoxyalkyl-amino-triazine d'un β-hydroxy-aldéhyde de la formule générale (II)

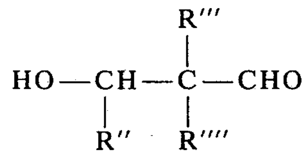

(II)

dans laquelle R'', R''' et R'''', qui peuvent être identiques ou différents, désignent chacun un atome d'hydrogène ou un radical alkyle en $C_1$ à $C_5$, R''' pouvant former avec l'atome de carbone $\alpha$ un noyau cyclopentane ou cyclohexane, R''' et (ou) R'''' pouvant en outre être un radical hydroxy-alkyle en $C_1$ à $C_4$ et R'''' aussi un groupe phényle, et élimination de l'alcool formé pendant la transéthérification.

2. Produits de transéthérification d'alcoxyalkylamino-triazines suivant la revendication 1, caractérisés en ce que l'alcoxyalkyl-amino-triazine est essentiellement de l'hexaméthoxyméthyl-amino-triazine.

3. Produits de transéthérification d'alcoxyalkylamino-triazines suivant la revendication 1 ou la revendication 2, caractérisés en ce que le β-hydroxy-aldehyde est l'aldéhyde hydroxy-pivalique.

4. Produits de transéthérification d'alcoxyalkylamino-triazines suivant l'une des revendications précédentes, caractérises en ce qu'ils ont été préparés par la réaction d'alcoxyalkyl-amino-triazines de la formule générale (I) et de 0,5 à 4,5 moles par mole d'alcoxyalkyl-amino-triazine d'un β-hydroxy-aldéhyde de la formule générale (II).

5. Utilisation de produits de transéthérification d'alcoxyalkyl-amino-triazines suivant l'une des revendications 1 à 4 dans des vernis.